(19) Europäisches Patentamt
European Patent Office
Office européen des brevets

(11) Veröffentlichungsnummer: **0 019 769**
**B1**

(12) # EUROPÄISCHE PATENTSCHRIFT

(45) Veröffentlichungstag der Patentschrift: **12.05.82**

(51) Int. Cl.³: **A 01 N 43/84**, A 01 N 43/46, A 01 N 43/40, A 01 N 43/36

(21) Anmeldenummer: **80102514.9**

(22) Anmeldetag: **08.05.80**

(54) **Fungizid, enthaltend ein 3-Cyclohexan-1-amino-propanderivat, Verfahren zu seiner Herstellung und seine Verwendung zur Bekämpfung von Pilzen.**

(30) Priorität: **25.05.79 DE 2921131**

(43) Veröffentlichungstag der Anmeldung: **10.12.80 Patentblatt 80/25**

(45) Bekanntmachung des Hinweises auf die Patenterteilung: **12.05.82 Patentblatt 82/19**

(84) Benannte Vertragsstaaten: **AT BE CH DE FR GB IT LI NL SE**

(56) Entgegenhaltungen: **FR-A-2 371 436**
**FR-A-2 389 606**

(73) Patentinhaber: **BASF Aktiengesellschaft, Carl-Bosch-Strasse 38, D-6700 Ludwigshafen (DE)**

(72) Erfinder: **Himmele, Walter, Dr., Eichenweg 14, D-6909 Walldorf (DE)**
Erfinder: **Pommer, Ernst-Heinrich, Dr., Berliner Platz 7, D-6703 Limburgerhof (DE)**

**EP 0 019 769 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Fungizid, enthaltend ein 3-Cyclohexan-1-amino-propanderivat, Verfahren zu seiner Herstellung und seine Verwendung zur Bekämpfung von Pilzen

Die vorliegende Erfindung betrifft ein Fungizid für den Pflanzenschutz, das die trans-Verbindung oder ihr Salz einer 3-(p-tertiär-Butylcyclohexyl)-2-methyl-propanverbindung enthält, die in 1-Stellung mit dem Stickstoffatom eines stickstoffhaltigen Heterocyclus verbunden ist.

Aus der DE-OS 2 752 135 ist es bekannt, daß 3-(4'-tert.-Butyl-cyclohexyl-1')-2-methyl-1-piperidino-propane und ihre Salze zur Bekämpfung von pflanzenpathogenen Pilzen verwendet werden können.

Die genannten Verbindungen liegen im Hinblick auf die beiden Substituenten am Cyclohexanring in 1,4-Stellung als cis-trans-Isomerengemische vor.

Es ist ferner bekannt, 3,5-Dimethyl-piperidinone-4 oder 3-p-tertiär-Butyl-cyclohexyl-2-methyl-1-morpholyl- oder 1-Piperidinyl-propan-Verbindungen als Fungizide zu verwenden (FR-2 389 606 und FR-2 371 436). In allen diesen Fällen liegen die Verbindungen als cis-trans-Isomerengemische vor. Ein Hinweis auf die speziellen Wirkungen der trans-Verbindungen läßt sich daraus nicht entnehmen.

Es wurde gefunden, daß die trans-Verbindungen der Formel

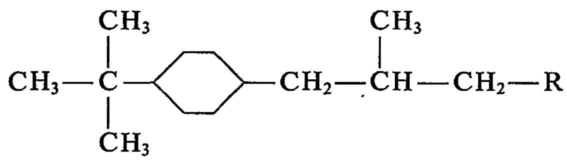

in der R den Rest eines am Stickstoff gebundenen gesättigten 5-, 6- oder 7gliedrigen stickstoffhaltigen heterocyclischen Restes bedeutet, der gegebenenfalls einfach oder mehrfach durch Methyl sustituiert ist und der gegebenenfalls in 4-Stellung zum Stickstoffatom anstelle des Methylenrestes ein Sauerstoffatom oder den C=O-Rest trägt, oder die Salze der trans-Verbindung eine bessere fungizide Wirkung als die entsprechende cis-Verbindung haben. Es ist überraschend, daß die trans-Verbindungen gegenüber Rostpilzen (Puccinia-Arten) an Getreide und Bohnen u. a. um das 10- bis 100fache stärker fungizid wirksam sind als die entsprechenden cis-Verbindungen. Unter der trans-Verbindung sind sowohl die reine trans-Verbindung zu verstehen als auch Gemische, die mehr als 50% (Gew.-%) der trans-Verbindung enthalten. Beispiele für den Rest R sind z. B. die Reste des Piperidins, Hexamethylenimins, 3,5-Dimethyl-4-piperidons und 2,6-Dimethylmorpholins. Salze sind beispielsweise die Salze mit Halogenwasserstoffsäuren, z. B. Salzsäure, Bromwasserstoffsäure, oder Salze mit Schwefelsäure, Essigsäure, p-Toluolsulfonsäure.

Die Bezeichnung »trans« bezieht sich auf die räumliche Stellung des tertiär-Butylrestes und des 2-Methyl-propylrestes am Cyclohexanring.

Die trans-Verbindungen werden erhalten, wenn man das bei der Hydrierung der entsprechenden 1,4-disubstituierten Phenylverbindung zur Cyclohexanverbindung erhaltene cis-trans-Gemisch nach dem Abdestillieren des für die Hydrierung verwendeten Lösungsmittels durch eine fraktionierte Destillation bei einem Druck am Kolonnenkopf von 0,05 bis 50 Torr an einer 10–100bödigen Kolonne unter einem Rücklaufverhältnis von 1 : 1 bis 1 : 100 durch Fraktionierung auf einen trans-Anteil von über 85% anreichert und das reine trans-Isomere nach Umwandlung in ein Säureadditionssalz an eine starke Säure und Kristallisation in einem organischen Lösungsmittel auskristallisieren läßt.

## Herstellungsverfahren

### 1) Herstellung von cis- und trans-3-(4-tert.-Butyl-cyclohexyl-1)-2-methyl-1-(3'-methyl-1-piperidino)-propan

a) Synthese des aromatischen Amins:
In einem 2-l-Dreihalskolben werden 306 g 3-(p-tert.-Butyl-phenyl)-2-methyl-propanal vorgelegt und 72,5 g 98%ige (Gew.-%) Ameisensäure zugetropft. Danach werden im Verlauf von drei Stunden 149 g 3-Methylpiperidin tropfenweise zugegeben. Die Temperatur erhöht sich dabei von 25 auf über 50° C. Anschließend wird das Reaktionsgemisch noch 12 Stunden am Rückfluß erhitzt, wobei $CO_2$-Entwicklung auftritt.
Nach beendeter Umsetzung wird unter vermindertem Druck fraktioniert destilliert. Nach einem Vorlauf von 47 g, der bis zu einer Temperatur von 130° C bei 0,2 Torr übergeht, destilliert das 3-(p-tert.-Butylphenyl)-2-methyl-1-(3'-methyl-1'-piperidino)-propan zwischen 130 bis 133° C bei einem Druck von 0,2 Torr über. Es werden 355 g erhalten, was einer Ausbeute, bezogen auf den eingesetzten Aldehyd, von 82,4% entspricht.

b) Hydrierung zum Cyclohexanderivat:
In einem 3-l-Rollautoklav werden 309 g 3-(p-tert.-Butylphenyl)-2-methyl-1-(3'-methyl-1'-piperidino)-propan in 1000 g Dioxan mit Hilfe von 0,5 g $Ru_2O_3$-Hydrat hydriert. Bei einem

Wasserstoffdruck von 100 bar und einer Temperatur von 120°C wird nach 3 Stunden kein Wasserstoff mehr aufgenommen. Anschließend wird bei 140°C und 120 bar $H_2$-Druck innerhalb von 12 Stunden ein Druckabfall von 35 bar festgestellt und nach Neueinstellen des Wasserstoffdruckes auf 160 bar wird bei einer Temperatur von 160°C im Verlauf von weiteren 10 Stunden nochmals ein Druckabfall von 10 registriert. Bei weiterem Anheben des Wasserstoffdrukkes auf 160 bar und der Temperatur auf 180°C tritt keine Wasserstoffaufnahme mehr ein.

c) Fraktionierte Destillation:
Das Reaktionsgemisch, 1310 g, wird über eine Siebbodenkolonne mit 10 Siebböden bei einem Druck von 10 mbar fraktioniert destilliert. Nach dem Abdestillieren des Dioxans werden bei einem Rücklaufverhältnis von 1 : 5 folgende Fraktionen erhalten:

1. bis 185°C 27 g enthaltend 85% cis- und 10% trans-Verbindung (Rest von 5% ist Vorlauf)
2. bei 185°C 114 g enthaltend 75% cis- und 25% trans-Verbindung
3. bis 186°C 60 g enthaltend 40% cis- und 60% trans-Verbindung
4. bis 187°C 55 g enthaltend 30% cis- und 70% trans-Verbindung

Der Destillationsrückstand von 40 g wird einer Kurzweg-Destillation bei 10 mbar und 187°C unterworfen. Es werden 38 g der Zusammensetzung 92% trans und nur 8% cis erhalten. Es bleiben 2 g Destillationsrückstand.
Das cis-trans-Verhältnis wird jeweils durch Gaschromatographie bestimmt.
504 g nach der oben beschriebenen Methode hergestelltes 3-(4'-tert.-Butyl-cyclohexyl-1'-)-2-methyl-1-(3'-methyl-1'-piperidino)-propan, die 54% cis- und 46% trans-Cyclohexanderivat enthalten (insgesamt 425 g) werden an einer Kolonne mit 20 Siebböden fraktioniert. Es wird ein Rücklaufverhältnis von 1 : 3 eingestellt.
Bis zu einer Übergangstemperatur von 192°C gehen 70 g Vorlauf über. Danach werden die folgenden Fraktionen erhalten:

1. bis 197°C/28 mbar 13 g enthaltend: 85% cis und 3% trans (Rest von 12% ist Vorlauf)
2. bis 198°C/26 mbar 52 g enthaltend: 93% cis und 7% trans
3. bis 197°C/25 mbar 87 g enthaltend: 90% cis und 10% trans
4. bis 192°C/22 mbar 82 g enthaltend: 85% cis und 15% trans
5. bis 196°C/22 mbar 81 g enthaltend: 80% cis und 20% trans
6. bis 202°C/28 mbar 87 g enthaltend: 30% cis und 70% trans
7. bis 196°C/22 mbar 73 g enthaltend: 97% trans und 3% cis.

Der Destillationsrückstand von 27 g wird einer Kurzweg-Destillation unterworfen. Es werden 24 g erhalten mit einem Gehalt an trans-Verbindung von über 98%.

d) Herstellung des HBr-Salzes der reinen cis-Form:
Die weitere Reinigung der Anfangs- und/oder Endfraktionen zu der reinen cis-Form wird durch Umkristallisation des Hydrobromids in Essigsäure/Wasser und im Falle der trans-Verbindung mit Hilfe von Chlorwasserstoff in Methanol vorgenommen:
25 g der oben erhaltenen ersten Fraktion werden mit 20 g wäßriger 48%iger Bromwasserstoffsäure versetzt. Danach werden 50 g Essigsäure zugegeben und das gesamte Kristallisat durch Erwärmen in Lösung gebracht. Beim Abkühlen kristallisiert das Hydrobromid der cis-Form aus. Die NMR-Analyse bestätigt das Vorliegen der reinen cis-Form. Es werden 28 g mit einem Schmelzpunkt von 210°C erhalten.

C ber.: 64,15% gef.: 64,5%; H ber.: 10,77%, gef.: 10,3%
N ber.: 3,74% gef.: 3,60%; Br ber.: 20,34%, gef.: 20,6%

e) Herstellung des HCl-Salzes der reinen trans-Form:
35 g der oben erhaltenen Fraktion 5 werden mit 60 g Chlorwasserstoff in Methanol versetzt. Beim Abkühlen kristallisieren 28 g Hydrochlorid mit einem Schmelzpunkt von 168°C aus. Die NMR-Analyse bestätigt das Vorliegen der trans-Form.

2) Herstellung von cis- und trans-3-(4'-tert.-Butyl-cyclohexyl-1')-2-methyl-1-(3',5'-dimethyl-1'-piperidino)-propan

a) 361 g 3-(p-tert.-Butylphenyl)-2-methyl-propanal werden, wie unter Beispiel 1 beschrieben, mit 200 g 3,5-Dimethyl-piperidin und 86 g 98%iger Ameisensäure umgesetzt. Die Reinigung des Amins

wird durch fraktionierte Destillation bei einem Druck von 0,2 mbar über eine 10-Bodenkolonne bewirkt. Bei 136° C gehen 406 g reines Amin über.

b) Hydrierung zur Cyclohexanverbindung:
In einem Schüttelautoklaven mit einem Reaktionsvolumen von 250 ml werden 70 g des 3-(p-tert.-Butylphenyl)-2-methyl-1-(3',5'-dimethyl-1'-piperidino)-propans mit 80 g Dioxan versetzt. Als Hydrierkatalysator werden 0,5 g Rutheniumoxidhydrat zugegeben. Bei 100 bar Wasserstoff und 120° C werden innerhalb von 37 Stunden 358 bar nachgepreßt.
Die fraktionierte Destillation des Reaktionsaustrages (139 g) liefert 64 g des Cyclohexanderivates, das zwischen 136 bis 146° C bei einem Druck von 3 Torr überdestilliert. Die gaschromatographische Analyse zeigt, daß es sich um ein Isomeren-Gemisch der beiden Formen cis und trans des Cyclohexanderivates handelt.

c) Fraktionierte Destillation:
562 g des aromatischen Amins, das in 1000 g Dioxan mit Hilfe von $Ru_2O_3$ · Hydrat bei 140 bar Wasserstoffdruck und bei 120°C innerhalb von 20 Stunden hydriert wurde, wird bei der Fraktionierung über eine Kolonne mit 10 Siebböden bei einem Rücklaufverhältnis von 1 : 5 getrennt, wie unter Beispiel 1 beschrieben.
Das über 90%ige cis-Cyclohexanderivat geht bei einem Druck von 0,1 Torr zwischen 124—125° C über. Das auf über 90% angereicherte trans-Cyclohexanderivat geht zwischen 129—131° C über.
Eine Wiederholung der fraktionierten Destillation über einer 80 cm langen mit Silberdrahtnetzwendeln bestückten Kolonne bei einem Druck von 12—13 Torr ermöglicht die Gewinnung von 154 g von auf über 90% angereicherten cis-Verbindung. Ein Zwischenlauf von 136 g bestand aus einem Gemisch von 1 : 1 cis-trans-Cyclohexanderivat. An trans-angereichertem Cyclohexanderivat (Anreicherung auf über 90%) werden 98 g gewonnen werden.
Der Zwischenlauf wird erneut zur Fraktionierung eingesetzt. Eine Rückstandsbildung trat nur in einem sehr geringen Umfang auf, bei der fraktionierten Destillation des cis-trans-Isomerengemisches bilden sich aus 390 g lediglich 2 g höhersiedende Anteile. Bei einem Druck von 12 Torr geht das cis-Derivat zwischen 193 bis 195° C und das angereicherte trans-Produkt zwischen 199—200° C über.

d) Kristallisation der reinen cis-Verbindung über das HCl-Salz:
20 g des auf über 90% angereicherten cis-Produktes werden in 25 g methanolischer Chlorwasserstofflösung aufgenommen, wobei sich die Lösung bis fast zum Sieden erhitzt. Beim Abkühlen kristallisieren 12 g Hydrochlorid aus (Wirkstoff Nr. 19). Der Schmelzpunkt des umkristallisierten cis-Cyclohexanderivats liegt bei 240° C. Eine Probe des Salzes, die mit KOH zerlegt wurde, zeigte bei der gaschromatographischen Analyse, daß es sich um trans-freie cis-Verbindung handelt.

e) Kristallisation der reinen trans-Verbindung über das HCl-Salz:
20 g der auf über 90% angereicherten trans-Verbindung werden in 30 g äthanolischer Chlorwasserstofflösung aufgenommen. Beim Abkühlen kristallisiert das Hydrochlorid der reinen trans-Verbindung aus vom Fp = 202° C (Wirkstoff Nr. 20). Es werden 13 g erhalten. Die freie Base, die durch Zerlegen einer Salzprobe erhalten wird, zeigt bei der gaschromatographischen Untersuchung, daß es sich um cis-freie trans-Verbindungen handelt. Die NMR-Analysen der beiden Kristallisate bestätigen die bereits durch die GC-Analyse erhaltenen Aussagen.

### 3) Herstellung von cis- und trans-3-(4'-tert.-Butylcyclohexyl-1')-2-methyl-1-(2',6'-cis-dimethyl-morpholyl)-propan

a) Die Herstellung des 3-(p-tert.-Butylphenyl)-2-methyl-1-(2',6'-cis-dimethyl-morpholyl)-propans erfolgt nach der unter Beispiel 1 beschriebenen Arbeitsweise durch Umsetzung von 3-(p-tert.-Butylphenyl)-2-methyl-propanal mit 2,6-cis-Dimethylmorpholin und Ameisensäure. Die Reinigung des aromatischen Amins erfolgt durch Destillation. Das Amin geht bei 3,0 mbar zwischen 170—175° C über.

b) Hydrierung zur Cyclohexanverbindung:
720 g des oben beschriebenen Morpholinderivates werden mit 500 g Dioxan versetzt. Als Hydrierkatalysator werden 0,5 g Rutheniumoxidhydrat zugegeben. Die Hydrierung wird bei einem Wasserstoffdruck von 120 bar und bei einer Temperatur von 140° C durchgeführt. Innerhalb von 24 Stunden werden bei der im 2,6-l-Rollautoklav durchgeführten Hydrierung insgesamt 130 bar Wasserstoff nachgepreßt.

c) Fraktionierte Destillation:
623 g des erhaltenen cis-trans-Isomerengemisches, das zu 60% aus cis- und 40% aus trans-Verbindung besteht, werden in einer 80 cm mit Silberdrahtnetzwendeln gefüllten Kolonne bei einem Druck von 3,0 mbar fraktioniert. Als Rücklaufverhältnis werden 1 : 5 eingestellt. Die cis-Verbindung geht zwischen 144 bis 145°C bei 3,0 Torr über. Die Anreicherung der cis-Verbindung in den ersten drei Fraktionen zu je etwa 70 g erfolgt auf über 90%. Die letzten drei

Fraktionen enthalten die angereicherte trans-Verbindung. 71 g, 54 g und 38 g. Der trans-Anteil liegt über 85%.

d) Kristallisation des reinen cis- und trans-HCl-Salzes:
Nach der unter Beispiel 1 und 2 beschriebenen Methode läßt sich das reine Hydrochlorid der cis-Verbindung mit äthanolischer Chlorwasserstofflösung gewinnen. Fp. 161° C (Wirkstoff Nr. 23).
Die reine trans-Verbindung bildet ein Hydrochlorid mit einem Schmelzpunkt von 199° C (Wirkstoff Nr. 24).
Die gaschromatographische Analyse, der aus beiden Salzen durch Umsetzung mit KOH hergestellten freien Basen bestätigt die Reinheit der Basen. Das so vorgereinigte cis-Cyclohexanderivat enthält weniger als 0,5% trans-Produkt; das trans-Produkt enthält weniger als 1% cis-Verbindung. Die NMR-Analyse bestätigt den Befund der GC-Analyse.
In entsprechender Weise werden die übrigen Wirkstoffe hergestellt.

Liste der Wirkstoffe

| Wirkstoff Nr. | R | | | Fp/Kp °C |
|---|---|---|---|---|
| 1 | Pyrrolidin | cis | Base | $Kp_{16}$ = 177 |
| 2 | Pyrrolidin | trans | Base | $Kp_{4}$ = 130 |
| 3 | Pyrrolidin | cis | HCl-Salz | Fp 170 |
| 4 | Pyrrolidin | trans | HCl-Salz | Fp 208 |
| 5 | Piperidin | cis | Base | $Kp_{10}$ = 180 |
| 6 | Piperidin | trans | Base | $Kp_{3}$ = 135 |
| 7 | 4-Methyl-piperidin | cis | Base | $Kp_{20}$ = 205–206 |
| 8 | 4-Methyl-piperidin | trans | Base | $Kp_{3}$ = 146–147 |
| 9 | Hexamethylenimin | cis | Base | $Kp_{0,2}$ = 151 |
| 10 | Hexamethylenimin | trans | Base | $Kp_{0,2}$ = 153 |
| 11 | 3,5-Dimethyl-4-piperidon | cis | Base | $Kp_{3}$ = 144 |
| 12 | 3,5-Dimethyl-4-piperidon | trans | Base | $Kp_{3}$ = 148 |
| 13 | 4-Äthyl-piperidin | cis | Base | $Kp_{3}$ = 157 |
| 14 | 4-Äthyl-piperidin | trans | Base | $Kp_{4}$ = 159 |
| 15 | 4-Äthyl-piperidin | cis | HCl-Salz | Fp 214 |
| 16 | 4-Äthyl-piperidin | trans | HCl-Salz | Fp 240 |
| 17 | 3,5-Dimethylpiperidin | cis | Base | $Kp_{18}$ = 195 |
| 18 | 3,5-Dimethylpiperidin | trans | Base | $Kp_{18}$ = 199 |
| 19 | 3,5-Dimethylpiperidin | cis | HCl-Salz | Fp 240 |
| 20 | 3,5-Dimethylpiperidin | trans | HCl-Salz | Fp 202 |
| 21 | cis-2,6-Dimethyl-morpholin | cis | Base | $Kp_{3}$ = 145 |
| 22 | cis-2,6-Dimethyl-morpholin | trans | Base | $Kp_{3}$ = 148 |
| 23 | cis-2,6-Dimethyl-morpholin | cis | HCl-Salz | Fp 161 |
| 24 | cis-2,6-Dimethyl-dimethylmorpholin | trans | HCl-Salz | Fp 199 |

Die Fungizide sind insbesondere geeignet zur Bekämpfung von Pflanzenkrankheiten, z. B. Erysiphe graminis (echter Mehltau) an Getreide, Erysiphe cichoriacearum (echter Mehltau) an Kürbisgewächsen, Podosphaera leucotricha an Äpfeln, Uncinula necator an Reben, Erysiphe polygoni an Bohnen, Sphaerotheca pannosa an Rosen, Microspaera querci an Eichen, Botrytis cinerea an Erdbeeren, Reben, Mycosphaerella musicola an Bananen, Puccinia-Arten (Rostpilze) an Getreide, Uromyces appendiculatus und U. phaseoli an Bohnen, Hemileia vastatrix an Kaffee und Rhizoctonia solani. Sie sind systematisch wirksam; sie werden sowohl über die Wurzeln als auch über die Blätter aufgenommen und im Pflanzengewebe transportiert.

Bei der Anwendung der Wirkstoffe zur Behandlung von Pflanzen gegen Pilzinfektionen liegen die Aufwandmengen zwischen 0,025 und 5 kg Wirkstoff/ha Fläche. Zum Oberflächenschutz von Bäumen

oder Früchten können die Wirkstoffe auch in Verbindung mit Kunststoffdispersionen 0,25%ig bis 5%ig, bezogen auf das Gewicht der Dispersionen, verwendet werden. Die Fungizide enthalten im allgemeinen zwischen 0,1 und 95 Gewichtsprozent Wirkstoff, vorzugsweise zwischen 0,5 und 90%.

Die Wirkstoffe können mit anderen bekannten Fungiziden gemischt werden. In vielen Fällen erhält man dabei eine Vergrößerung des fungiziden Wirkungsspektrums; bei einer Anzahl von Fungizidmischungen in den Gewichtsverhältnissen 1 : 10 bis 10 : 1 treten auch synergistische Effekte auf, d. h., die fungizide Wirksamkeit des Kombinationsproduktes ist größer als die der addierten Wirksamkeiten der Einzelkomponenten. Fungizide, die mit den erfindungsgemäßen Verbindungen kombiniert werden können, sind beispielsweise:

Dithiocarbamate und deren Derivate, wie

Zinkdimethyldithiocarbamat,
Manganäthylenbisdithiocarbamat,
Mangan-Zink-äthylendiamin-bis-dithiocarbamat,
Zinkäthylenbisdithiocarbamat,
Tetramethylthiuramdisulfid,
Ammoniak-Komplex von Zink-(N,N-äthylen-bis-dithiocarbamat) und
N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;
heterocyclische Verbindungen, wie
N-Trichlormethylthio-tetrahydrophthalimid,
N-Trichlormethylthio-phthalimid,
N-(1,1,2,2-Tetrachloräthylthio)-tetrahydrophthalimid,
1-(Butylcarbamoyl)-2-benzimidazol-carbaminsäuremethylester,
2-Methyloxycarbonylamino-benzimidazol,
2,3-Dihydro-5-carboxanilido-6-methyl-1,4-oxathiin-4,4-dioxid,
2,3-Dihydro-5-carboxanilido-4-hydroxy-6-methyl-pyrimidin,
5-Butyl-2-dimethylamino-4-hydroxy-6-methyl-pyrimidin,
1,2-Bis-(3-äthoxycarbonyl-2-thioureido)-benzol,
1,2-Bis-(3-methoxycarbonyl-2-thioureido)-benzol und verschiedene andere Fungizide, wie
Dodecylguanidinacetat,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Jodbenzoesäureanilid,
2-Brombenzoesäureanilid,
3-Nitro-isophthalsäure-diisopropylester,
1-(1,2,4-Triazolyl-1')-[1-(4'-chlorphenoxy)]-3,3-dimethylbutan-2-on,
1-(1-Imidazolyl)-2-allyloxy-2-(2,4-dichlorphenyl)-äthan,
Piperazin-1,4-diyl-bis-1-(2,2,2-Trichloräthyl)-formamid,
2,4,5,6-Tetrachlor-isophthalonitril,
1,2-Dimethyl-3,5-diphenyl-pyrazoliniummethylsulfat.

Die Anwendung erfolgt z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen, Beizen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z. B. Dimethylformamid, Dimethylsulfoxid, N-Methylpyrrolidon, Wasser usw. in Betracht. Die Salze können in Form ihrer wäßrigen Lösungen verwendet werden.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung

mit Wasser geeignet sind.

An oberflächenaktiven Stoffen sind zu nennen:

Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther. Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Zu den Mischungen oder Einzelwirkstoffen können Öle verschiedenen Typs, Herbizide, Fungizide, Nematozide, Insektizide, Bakterizide, Spurenelemente, Düngemittel, Antischaummittel (z. B. Silikone), Wachstumsregulatoren, Antidotmittel oder andere wirksame Verbindungen, zugesetzt werden.

## Versuch 1

### Wirksamkeit gegen Weizenmehltau

Blätter von in Töpfen gewachsenen Weizenkeimlingen der Sorte »Jubilar« werden mit wäßrigen Emulsionen aus 80% (Gewichtsprozent) Wirkstoff und 20% Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

| Wirkstoff | Befall der Blätter nach Spritzungen mit x%iger Wirkstoffbrühe | | | |
|---|---|---|---|---|
| | x = 0,006 | 0,003 | 0,0015 | 0,0007 |
| 9 (cis-Form) | 0 | 2 | 3 | 3 |
| 10 (trans-Form) | 0 | 0 | 0 | 0 |
| 23 (cis-Form) | 0 | 2 | 2–3 | 2–3 |
| 24 (trans-Form) | 0 | 0 | 0 | 0 |
| Kontrolle (unbehandelt) | 5 | | | |

0 = kein Befall, abgestuft bis 5 = Totalbefall.

## Versuch 2

### Wirksamkeit gegen Weizenbraunrost

Blätter von in Töpfen gewaschenen Weizenpflanzen werden mit Sporen des Weizenbraunrostes (Puccinia recondita) künstlich infiziert und 48 Stunden lang bei 20 bis 25°C in einer

wasserdampfgesättigten Kammer aufgestellt. Danach werden die Pflanzen mit wäßrigen Spritzbrühen, die in dem Wasser gelöst oder emulgiert eine Mischung aus 80% des zu prüfenden Wirkstoffes und 20% Natriumligninsulfonat enthalten, besprüht und im Gewächshaus bei Temperaturen zwischen 20 und 22°C und bei 75 bis 80% relativer Luftfeuchtigkeit aufgestellt. Nach 10 Tagen wird das Ausmaß der Rostpilzentwicklung beurteilt.

| Wirkstoff | Befall der Blätter nach Spritzungen mit x%iger Wirkstoffbrühe | | | |
|---|---|---|---|---|
| | x = 0,012 | 0,006 | 0,003 | 0,0015 |
| 5 (cis-Form) | 2–3 | 2–3 | 2–3 | 4 |
| 6 (trans-Form) | 0 | 0 | 0 | 3 |
| 11 (cis-Form) | 2 | 2–3 | 4 | 4–5 |
| 12 (trans-Form) | 0 | 0 | 0 | 2 |
| 21 (cis-Form) | 2 | 3 | 3 | 4 |
| 22 (trans-Form) | 0 | 0 | 0 | 0 |
| 23 (cis-Form) | 3 | 5 | 5 | 5 |
| 24 (trams-Form) | 0 | 0 | 0 | 2 |
| Kontrolle (unbehandelt) | 5 | | | |

0 = kein Befall, abgestuft bis 5 = Totalbefall.

### Beispiel 1

Man vermischt 90 Gewichtsteile der Verbindung 10 mit 10 Gewichtsteilen N-Methyl-α-pyrrolidon und erhält eine Lösung, die zur Anwendung in Form kleinster Tropfen geeignet ist.

### Beispiel 2

20 Gewichtsteile der Verbindung 6 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-mono-äthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

### Beispiel 3

20 Gewichtsteile der Verbindung 12 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteile Wasser erhält man eine wäßrige Dispersion, die 0,02 Gewichtsprozent des Wirkstoffs enthält.

### Beispiel 4

20 Gewichtsteile der Verbindung 22 werden in einer Mischung gelöst, die aus 25 Gewichtsteilen Cyclohexanol, 65 Gewichtsteilen einer Mineralölfraktion vom Siedepunkt 210 bis 280°C und 10

Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 5

20 Gewichtsteile des Wirkstoffs 6 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 6

3 Gewichtsteile der Verbindung 12 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 7

30 Gewichtsprozent der Verbindung 22 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 8

40 Gewichtsteile des Wirkstoffs 6 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoffe enthält.

Beispiel 9

20 Teile des Wirkstoffs 4 werden mit 12 Teilen Calciumsalz der Dodecylbenzolsulfonsäure, 8 Teile Fettalkohol-polyglykoläther, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

**Patentansprüche**

1. Fungizid für den Pflanzenschutz, enthaltend eine trans-Verbindung der Formel

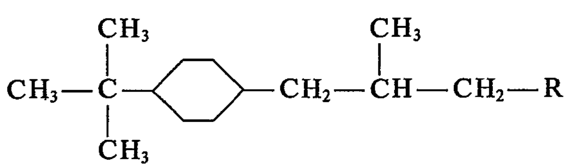

in der R den Rest eines am Stickstoff gebundenen gesättigten 5-, 6- oder 7gliedrigen stickstoffhaltigen heterocyclischen Restes bedeutet, der gegebenenfalls einfach oder mehrfach durch Methyl substituiert ist und der gegebenenfalls in 4-Stellung am Stickstoffatom anstelle des Methylenrestes ein Sauerstoffatom oder den C=O-Rest trägt, oder die Salze der trans-Verbindung.

2. Fungizid für den Pflanzenschutz, enthaltend einen festen oder flüssigen Trägerstoff und eine trans-Verbindung wie im Anspruch 1 definiert.

3. Verfahren zur Herstellung eines Fungizids für den Pflanzenschutz, dadurch gekennzeichnet, daß man einen festen oder flüssigen Trägerstoff vermischt mit einer trans-Verbindung wie im Anspruch 1 definiert.

4. Verfahren zur Bekämpfung von Pilzen im Pflanzenschutz, dadurch gekennzeichnet, daß man die

Pilze oder die vor Pilzbefall zu schützenden Pflanzen behandelt mit einer trans-Verbindung wie im Anspruch 1 definiert.

5. Fungizid für den Pflanzenschutz gemäß Anspruch 1, enthaltend das trans-3-(4′-tertiär-Butyl-cyclohexyl-1′)-2-methyl-1-(2′,6′-dimethyl-morpholino)-propan.

## Claims

1. A fungicide for crop protection, containing a trans commpound of the formula

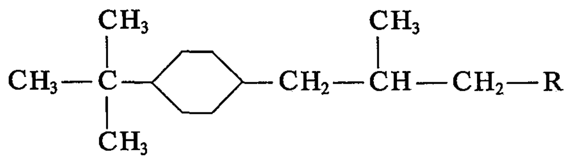

where R denotes the radical of a saturated, 5-, 6- or 7-membered nitrogenous heterocyclic radical which is attached at the nitrogen, is unsubstituted or mono- or polysubstituted by methyl, and may bear, in the 4-position to the nitrogen atom, an oxygen atom or a C=O radical instead of the methylene radical, or a salt thereof.

2. A fungicide for crop protection, containing a solid or liquid carrier and a trans compound as defined in claim 1.

3. A process for the manufacture of a fungicide for crop protection, characterized in that a solid or liquid carrier is mixed with a trans compound as defined in claim 1.

4. A process for combating fungi in the crop protection field, characterized in that the fungi or the plants to be protected against fungus attack are treated with a trans compound as defined in claim 1.

5. A fungicide for crop protection, as claimed in claim 1, containing trans-3-(4′-tert-butylcyclohexyl-1′)-2-methyl-1-(2′,6′-dimethylmorpholino)-propane.

## Revendications

1. Fongicide phytosanitaire, contenant un composé trans de la formule

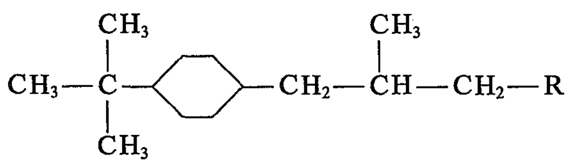

dans laquelle R désigne le reste d'un groupe hétérocyclique azoté penta-, hexa- ou heptagonal saturé, fixé à l'atome d'azote, éventuellement substitué par un ou plusieurs radicaux méthyle et comportant éventuellement en position 4 par rapport à l'atome d'azote, au lieu d'un pont méthylène, un atome d'oxygène ou un groupe C=O-, ou des sels d'un tel composé trans.

2. Fongicide phytosanitaire, contenant un support ou véhicule solide ou liquide et un composé trans tel que défini dans la revendication 1.

3. Procédé de préparation d'un fongicide phytosanitaire, caractérisé en ce que l'on mélange un support ou véhicule solide ou liquide avec un composé trans tel que défini dans la revendication 1.

4. Procédé pour combattre les champignons dans le règne végétal, caractérisé en ce que l'on traite les champignons ou les plantes à protéger des champignons avec un composé trans tel que défini dans la revendication 1.

5. Fongicide phytosanitaire suivant la revendication 1, contenant du 3-(4′-tertiobutyl-cyclohexyl-1′)-2-méthyl-1-(2′,6′-diméthyl-morpholino)-propane trans.